# FASCICULE DE BREVET EUROPEEN

(11) **EP 0 253 702 B1**
(45) Date de publication et mention de la délivrance du brevet: **12.05.1993**
(21) Numéro de dépôt: 87401443.4
(22) Date de dépôt: 23.06.1987
(51) Int. Cl.: C05F 9/02, B01J 19/00, B01J 19/28, C12M 1/36

(54) **Procédé et dispositif pour le traitement des déchets dans un appareil de fermentation accélérée**
Verfahren und Einrichtung zur Behandlung von Abfallstoffen in einer Schnellgärungsvorrichtung
Method and apparatus for the treatment of waste in an apparatus for accelerated fermentation

(30) Priorité: 23.06.1986 FR 8609037; 03.10.1986 FR 8613844
(43) Date de publication de la demande: 20.01.1988
(73) Titulaire: T.E.S. S.A., F-42153 Riorges (FR)
(72) Inventeur: Royal, Gabriel, F-42300 Villerest (FR)
(74) Mandataire: Rodhain, Claude

(56) Documents cités:
- BE-A- 555 099
- BE-A- 560 216
- DE-A- 1 963 484
- DE-C- 941 120
- DE-C- 3 442 058
- FR-A- 1 426 317
- FR-A- 1 567 550
- FR-A- 2 195 334
- FR-A- 2 303 776
- FR-A- 2 482 582
- GB-A- 1 323 676
- US-A- 2 551 774
- US-A- 2 994 592
- US-A- 3 149 922
- US-A- 3 248 176
- US-A- 3 930 799

## Description

La présente invention concerne un dispositif de traitement des déchets, notamment d'ordures ménagères.

Les dispositifs de traitement de déchets, notamment pour ordures ménagères, sont des appareils de forme générale de révolution à axe de rotation sensiblement horizontal. Les déchets sont introduits à l'intérieur de l'appareil par l'une des extrémités. Le traitement consiste à faire tourner cet appareil à une vitesse très lente de l'ordre de quelques tours par minute. La paroi de l'appareil comporte généralement des dents qui déchirent les sacs plastiques pour libérer leur contenu et qui assurent un mélange efficace lors de la rotation. Des réactions bactériologiques et enzymatiques assurent une autofermentation aérobie des matières organiques et les calories dégagées par cette réaction exothermique provoquent une élévation de la température qui stérilise le milieu à condition que le minimum d'élévation de la température et le minimum de durée du maintien en température soient respectés.

Ces dispositifs ont, par ailleurs, l'avantage de ne pas briser les verres en éclats de trop petites dimensions et de ne pas pulvériser les plastiques, ces matériaux pouvant donc être séparés du compost par simple criblage en fin de traitement. Le compost, issu du traitement dans cet appareil, doit répondre à certains critères de qualité ; or l'alimentation tout comme le type des déchets introduits varient.

De même, l'alimentation n'est pas régulière et la quantité de produit à l'intérieur du tube varie également. L'appareil utilisé est supporté, comme décrit plus bas, par des ensembles de supportage montés mobiles sur un système à compensation de pression et disposés le long de l'axe de révolution, l'ensemble étant géré par une centrale de pilotage. Ainsi, les déformations engendrées par les variations de poids du produit sont corrigées.

La fermentation est régulée par un procédé de pilotage selon lequel on contrôle la température, le degré hygrométrique et la quantité du produit par la détermination de son profil de façon continue et directement sur l'appareil en rotation.

Selon ce procédé, la température est mesurée périodiquement et directement dans le produit.

Le degré hygrométrique est déterminé par échantillonnage des gaz lorsque la prise d'aspiration est dégagée du produit. Le degré hygrométrique peut aussi être déterminée par la mesure de la résistivité du produit effectuée dans la masse du produit.

Le profil est obtenu par localisation du point d'intersection de la surface libre du produit avec la paroi de l'appareil à l'aide d'une détection acoustique du choc des particules les plus grosses qui roulent sur la masse du produit, la surface étant inclinée par le mouvement de rotation. On déduit alors la quantité de produit.

Une autre caractéristique concerne l'échange d'information réalisé par un microprocesseur embarqué qui collecte les informations et les transmet à la centrale de pilotage.

De même l'information en retour qui comporte tous les ordres est renvoyée au microprocesseur qui agit sur les différents paramètres.

Ainsi, des sources de fluide sont reliées à des vannes de distribution montées sur l'appareil par des raccords tournants, ces vannes étant pilotées par le microprocesseur.

Les appareils comme décrits ci-dessus et utilisés dans divers domaines de l'industrie nécessitent d'avoir un supportage efficace et un dispositif d'entraînement pour la mise en rotation autour de l'axe de révolution. La surface externe du matériel repose généralement sur deux paires de galets par l'intermédiaire de deux bandes de roulement solidaires de cette surface externe. L'entraînement est assuré par une couronne dentée entraînée par un pignon solidaire de l'axe d'un moteur plus particulièrement un moteur électrique. La longueur de ces appareils est de quelques dizaines de mètres, les diamètres extérieurs pouvant atteindre 4 à 5 mètres. L'intérieur de ces appareils est muni suivant l'utilisation, d'un matériau réfractaire de four, de spirales, d'ailettes de mélanges dans le cas de broyeur, ou bien de tôles perforées dans le cas des cribles. Le poids résultant de ces appareils est donc très élevé.

Le document US-A-2 551 774 décrit un cylindre rotatif à axe de rotation sensiblement horizontal dans lequel la charge peut varier suivant la longueur, et qui comprend plusieurs dispositifs de supportage repartis le long de l'axe dudit cylindre. Chaque dispositif de supportage comprend un moyen de pression qui agit continuellement avec une force prédéterminée et uniforme sur le cylindre rotatif. La force est réglée telle qu'elle est légèrement inférieure à la charge que le dispositif de supportage correspondant doit normalement supporter.

La construction de tels appareils généralement de forme cylindrique est obtenue, par liaison d'un ensemble de tronçons de cylindre aboutés et soudés les uns aux autres. Les deux chemins de roulement sont alors rapportés sur ce cylindre ainsi que la couronne d'entraînement. Afin de ne pas introduire de contraintes différentielles le long du tube notamment par un appui sur trois points au lieu de quatre, il est nécessaire que ces deux chemins de roulement aient un diamètre parfaitement identique et que leur concentricité soit parfaite ainsi que leur alignement. De même la couronne d'entraînement nécessite un montage particulier effectué à l'aide d'un dispositif de pré-contrainte permettant de tenir compte des efforts réactifs très importants du pignon d'entraînement avec cette couronne lors du fonctionnement.

De telles réalisations conduisent à utiliser pour les besoins de résistance mécanique, des tôles de très forte épaisseur et il s'avère que même en faisant varier cette épaisseur en fonction du profil la masse finale est excessivement élevée et les réalisations très coûteuses.

Le fonctionnement de ces appareils du type cylindre rotatif est généralement continu mais la charge de travail varie en permanence suivant la quantité de produits issus de l'alimentation, de la quantité de produits évacués, ou plus simplement de la vitesse de rotation du cylindre et de son inclinaison. Aussi, de tels cylindres ont une tendance à la déformation notamment par flexion entre les deux dispositifs de supportage à galets et sous l'effet des efforts réactifs de ces galets. De façon à minimiser les déformations qui conditionnent la durée de vie de l'appareil les dimensionnements sont calculés avec de forts coefficients multiplicateurs. Par ailleurs, ces cylindres constitués d'une suite de tronçons liés les uns aux autres et renforcés éventuellement par des viroles, possèdent un très fort moment d'inertie dû à la forte épaisseur, et conduisent à des déformations par torsion autour de l'axe de rotation. De même le dispositif de mise en rotation demande des couples d'entraînement très élevés et un parfait ajustage des dentures du pignon et de la couronne outre les dispositifs de précontrainte déjà mentionnés.

Des perfectionnements ont conduit à réaliser la liaison du chemin de roulement et de la structure cylindrique par un montage sur clavettes de façon à autoriser un déplacement différentiel suffisant entre le chemin de roulement et la surface externe du cylindre. Un tel montage impose néanmoins des recalages fréquents et ne solutionne que le problème de la destruction de la soudure entre le chemin de roulement et le tube.

Un exemple de réalisation d'un tel appareil type incinérateur d'une longueur de 40 mètres, conduit à utiliser des tôles dont l'épaisseur varie de 0,018 mètre pour les parties les moins chargées à 0,035 mètre pour les parties les plus chargées.

Dans le but de remédier à ces divers inconvénients, la présente invention propose un dispositif de supportage et de mise en rotation d'un appareil de forme générale de révolution à axe de rotation sensiblement horizontal, d'une grande longueur par rapport au diamètre constitué de tronçons aboutés, appareil dans lequel la charge peut varier suivant la longueur, ce dispositif de supportage et de mise en rotation étant caractérisé en ce qu'il comprend au moins deux ensembles de supportage disposés le long de l'axe de révolution et montés mobiles sur un système à compensation de pression, au moins deux dispositifs d'entraînement, au moins un capteur de déplacement associé à chaque ensemble de supportage et une centrale de pilotage.

Ce supportage est en outre caractérisé en ce qu'il comprend au moins deux galets montés sur un système à compensation de pression de façon à équilibrer en permanence la force exercée sur le galet par un effort résistant strictement équivalent. Un tel système à compensation utilise des vérins hydrauliques, pneumatiques ou électro-mécaniques.

Le dispositif d'entraînement selon l'invention est constitué d'au moins un moteur et d'une chaîne d'entraînement, plusieurs de ces dispositifs d'entraînement étant répartis sur toute la longueur de l'appareil. Un galet tendeur est prévu sur chacun de ces dispositifs, il sera solidaire d'un système permettant de régler en fonction de la charge l'effort de pression tenant ainsi compte des efforts éventuels de répulsion.

Un tel dispositif permet de diminuer fortement la masse de l'appareil et par la même son inertie . Cette répartition permet d'utiliser des matériels et des matériaux beaucoup plus courants et évite tout sur-dimensionnement inutile. La flexion résultante peut être considérée comme négligeable et les efforts de torsion quasiment nuls. De même, l'épaisseur de la paroi de l'appareil restera constante sur toute la longueur. En effet, un tel système de supportage permet de compenser toute variation de charge sur la longueur de l'appareil.

L'invention va maintenant être décrite en détails en accord avec les figures annexées de façon à faire apparaître d'autres caractéristiques et avantages de l'invention.
- Figure 1 :: Dispositif de mesure de la température en coupe
- Figure 2 :: Dispositif de mesure du degré hygrométrique en coupe
- Figure 3 :: Liaisons fluides
- Figure 4 :: Dispositif acoustique pour la détermination du profil du produit dans l'appareil
- Figure 5 :: Synoptique de fonctionnement
- Figure 6 :: Vue longitudinale du dispositif selon l'invention;
- Figure 7 :: Coupe transversale du dispositif selon AA-AA;
- Figure 8 :: Coupe transversale du dispositif selon BB-BB.

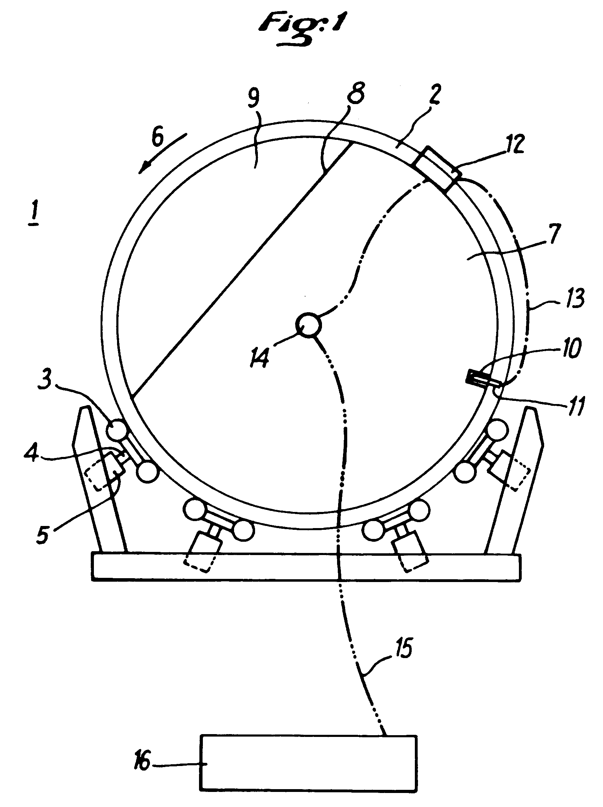

Sur la figure 1 on a représenté une coupe du cylindre de fermentation 1 qui fait apparaître la paroi 2 en appui par sa surface extérieure sur des galets tels que 3 intégrés dans un ensemble de supportage 4 monté mobile sur un système à compensation de pression 5. Ces ensembles de supportage sont multiples pour une même section et sont également répartis dans la direction longitudinale à intervalles réguliers. Le sens de rotation est indiqué par la flèche 6 et le produit est référencé en 7. Sa surface libre 8 est inclinée et laisse subsister un volume de gaz 9. De manière à assurer un bon mélange et à déchirer les sacs plastiques afin qu'ils se vident de leur contenu le cylindre est muni sur sa surface intérieure de dents 10 qui peuvent être du type pyramide creuse avec un sommet tronqué et comportant des ouvertures latérales. Il est alors possible d'introduire une sonde de température intrusive de façon à ce qu'elle débouche dans le volume interne de la dent. La sonde est ainsi protégée mécaniquement tout en restant périodiquement au contact du compost en formation.

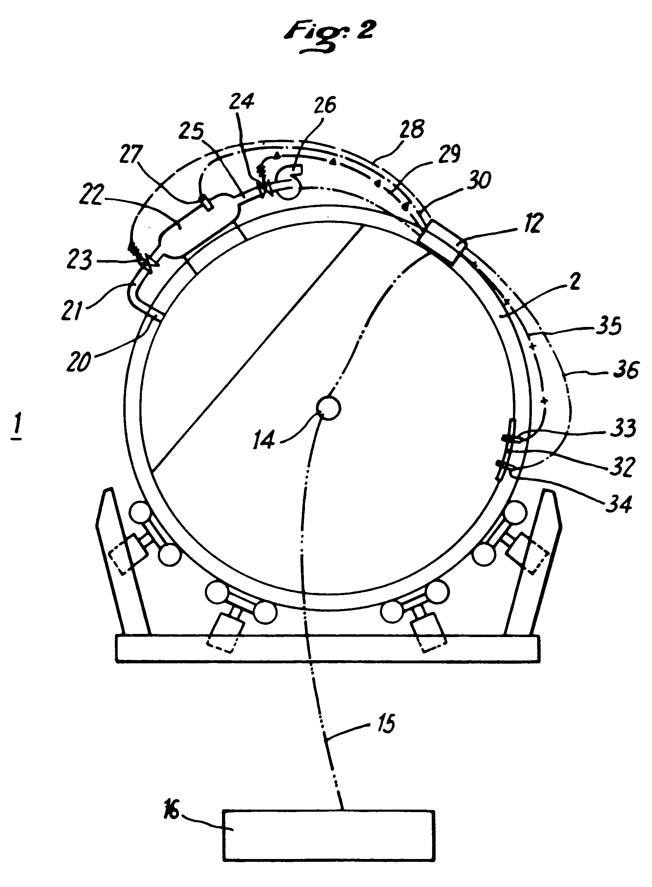

Les informations obtenues sont transmises par une liaison fixe 13 jusqu'au microprocesseur 12 disposé à l'extérieur du cylindre mais solidaire de celui-ci. Les informations du microprocesseur 12 sont à leur tour transmises à l'aide d'un coupleur rotatif 14 à la centrale de pilotage 16 par la liaison 15. La mesure du degré hygrométrique est effectuée dans un premier temps par échantillonnage de gaz tel que représenté sur la figure 2.

La paroi 7 est percée pour introduire une prise d'aspiration 20. Le tube 21 conduit le gaz jusqu'à une enceinte 22 isolable grâce à deux électrovannes 23 et 24. La conduite 25 se prolonge au-delà de l'électrovanne 24 par un ventilateur d'extraction 26. Une sonde 27 de mesure d'humidité est disposée de façon intrusive sur l'enceinte 22 pour être au contact du gaz. Des liaisons 28, 29, 30, 31 relient respectivement les électrovannes 23 et 24, la sonde 27 et le ventilateur 26 au microprocesseur 12. Ce dernier est relié à son tour à la centrale de pilotage 16 comme indiqué ci-dessus.

La mesure peut aussi être effectuée par détermination de la résistivité du produit lui-même. Un support 32 est alors fixé sur la surface interne du cylindre. Sur ce support sont disposées deux électrodes 33 et 34 dont l'écartement est connu. Chacune de ces électrodes est reliée au microprocesseur par des liaisons telles que 35, 36 de façon à ce que la mesure de la résistivité du produit soit transmise à ce microprocesseur.

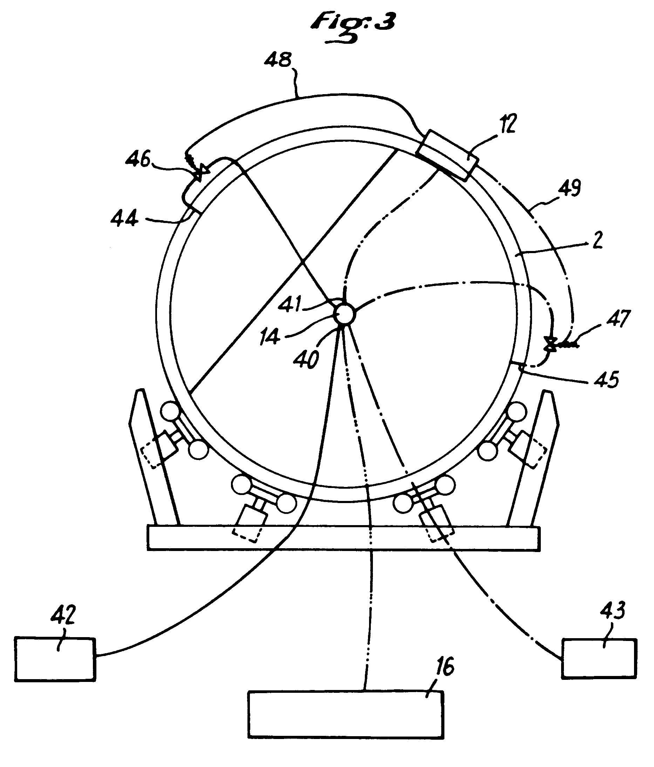

La figure 3 représente les adductions de fluide telles que air et eau par exemple. Le coupleur rotatif 14 est complété par deux coupleurs rotatifs 40 et 41 disposés respectivement entre les sources d'eau 42 et d'air 43 tous deux sous pression. Ces fluides sont introduits dans l'enceinte par des piquages 44 et 45. L'ouverture et la fermeture de ces piquages sont réalisées par deux électrovannes 46, 47 reliées électriquement au microprocesseur 12 par les liaisons 48 et 49.

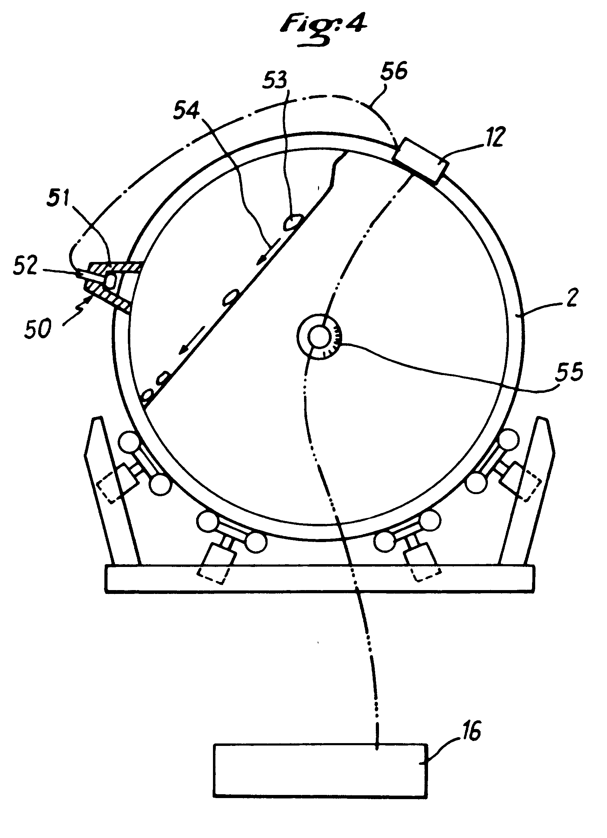

Sur la figure 4 on voit le moyen de détection acoustique 50 constitué d'une enceinte 51 et d'un microphone 52. L'enceinte est totalement hermétique et isolante, elle est par ailleurs fixée à l'extérieur de la paroi 2. Les particules les plus grosses 53 roulement sur la surface libre du produit comme indiqué par les flèches 54. Un codeur angulaire 55 associé donne à tout instant la position exacte du moyen de détection acoustique par rapport à un point origine. Par ailleurs, ce moyen de détection acoustique est relié au microprocesseur 12 par la liaison 56.

Ces groupes de dispositifs de mesure sont multiples et régulièrement répartis selon l'axe longitudinal du cylindre de façon que l'on puisse établir un profil selon cet axe des variations de chacun des paramètres. De façon simplificatrice nous ne nous intéressons dans le cadre de cette description qu'à un seul groupe de dispositif.

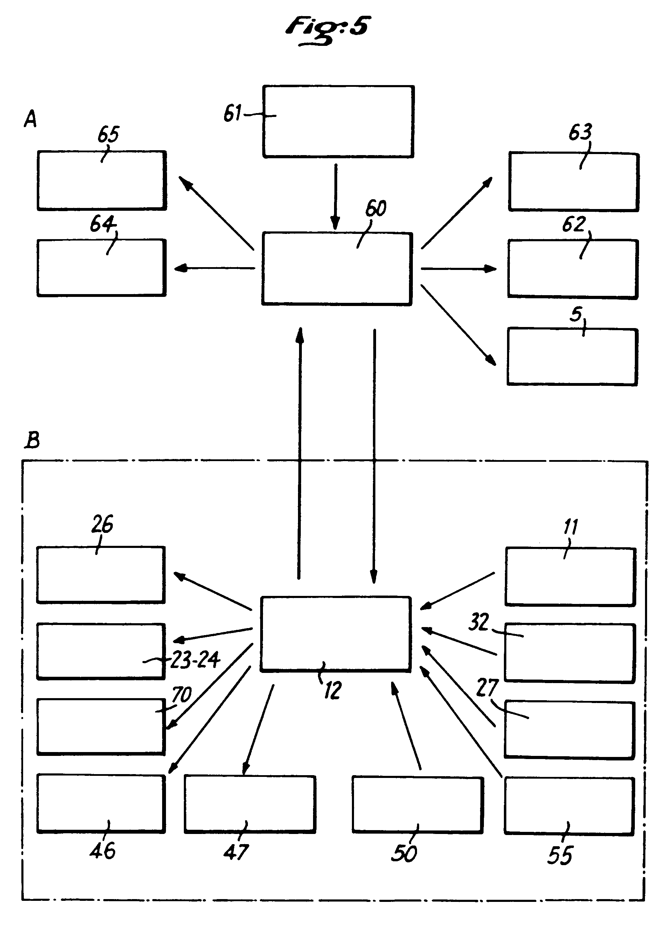

Le synoptique de la figure 5 est divisé en deux parties A et B suivant que les moyens sont disposés en salle de commande ou bien embarqués sur le cylindre de fermentation.

La centrale de pilotage est représentée en 60. Elle reçoit les ordres directement de l'opérateur 61 et les informations en provenance du microprocesseur 12 de la partie B.

Les ordres de l'opérateur sont rares et n'interviennent généralement que lors des phases transitoires de démarrage et d'arrêt de l'installation ou en cas d'incidents. Les informations reçues du microprocesseur 12 sont de différentes natures. Le codeur angulaire 55 donne la position du cylindre en rotation afin de déterminer l'instant de la prise de mesure de chacun des paramètres en fonction des nécessités.

Par exemple les sondes de température 11 et de résistivité 32 ne doivent être utilisées que lorsqu'elles sont plongées au sein du produit. Les informations ne sont transmises qu'à cet instant. La sonde de mesure d'humidité 27 n'est utilisée que périodiquement. Lorsque la prise d'aspiration 20 est dégagée du produit le ventilateur d'extraction 26 reçoit une impulsion, les électrovannes 23 et 24 étant ouvertes, pour introduire un échantillon de gaz dans l'enceinte 22 puis celle-ci est à nouveau isolée par les mêmes électrovannes. C'est à cet instant que la mesure est déclenchée puis transmise jusqu'au microprocesseur 12. On peut également grouper plusieurs échantillons et n'effectuer les mesures que tous les n tours.

Compte-tenu de ces mesures retransmises à la centrale de pilotage, le microprocesseur 12 reçoit des ordres pour commander à son tour l'admission contrôlée de fluides de correction par l'intermédiaire des électrovannes 46 et 47. Le moyen de détection acoustique 50 reçoit constamment les échos des chocs du produit contre la surface du cylindre à l'endroit où il se situe. Lorsque le cylindre tourne sur son axe, sa surface libre s'incline jusqu'au moment où le produit s'écoule selon sa pente naturelle, les éléments les plus lourds roulent et descendent plus vite que les éléments plus légers. Il y a ainsi choc de ces éléments lourds en un point particulier dont la position est déterminée en combinant le pic d'intensité sonore maximum et l'indication du codeur. On en déduit l'angle que fait la surface libre avec la verticale. Cette information permet à son tour de calculer le taux de remplissage et de commander l'introduction de déchets ou la vidange du compost dans l'appareillage. Ces fonctions sont regroupées en 70, et les dispositifs classiques ne sont pas représentés.

Les capteurs de position 62 (non représentés) associés aux groupes de supportage effectuent des mesures différentielles des déformations du cylindre et ces informations sont également utilisées par la centrale de pilotage pour commander l'admission ou la vidange du fluide des systèmes à compensation de pression 5.

Les variations de la vitesse de rotation déterminées par la centrale de pilotage sont représentées en 63. L'affichage des informations est classique. Les références 64 et 65 concernent respectivement une visualisation à l'aide d'un synoptique lumineux ou d'une imprimante.

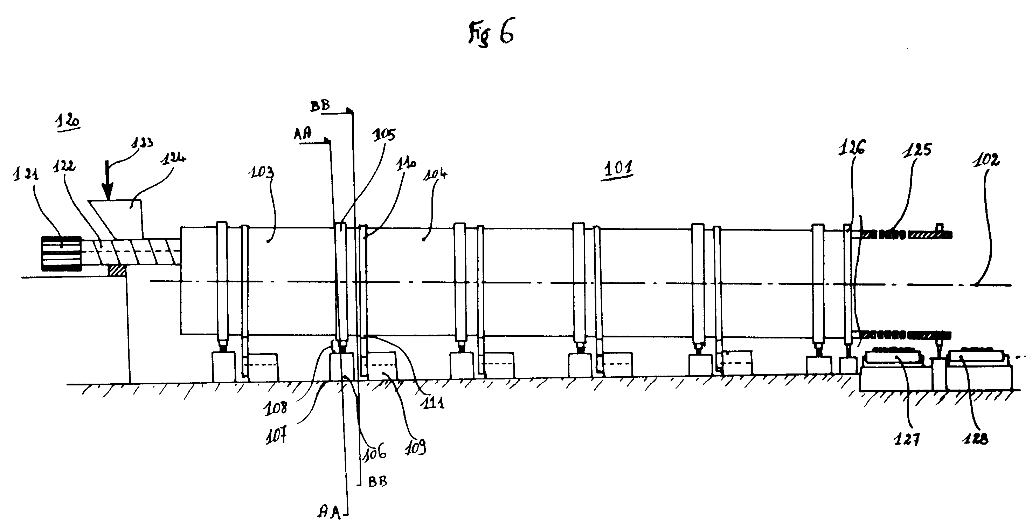

Sur la figure 6, on voit un appareil 101 selon l'invention de forme générale de révolution pouvant tourner autour d'un axe 102 sensiblement horizontal. Le cylindre qui constitue cet appareil est réalisé par aboutement de plusieurs tronçons cylindriques tels que 103 et 104 aboutés et assemblés par soudure. Une virole telle que 105 située entre les deux tronçons 103 et 104 facilite l'opération de liaison par soudage et renforce cette partie plus fragile. Par ailleurs, cette virole 105 est utilisée comme chemin de roulement. En effet, l'appareil 101 repose sur des ensembles de supportage tels que 106 qui prennent appui sur le sol en 107 et restent en contact avec le chemin de roulement 105, par l'intermédiaire d'un dispositif à galets 108 qui sera décrit plus en détail ultérieurement. Le cylindre est entraîné en rotation par un moteur hydraulique 109 et une chaîne 110 associée en prise sur une couronne 111 solidaire de la paroi externe de la partie cylindrique 104. Ce module constitué de deux tronçons aboutés et liés entre eux, renforcé par une virole qui assure la fonction de chemin de roulement, d'un ensemble de supportage et d'un dispositif d'entraînement peut être répété autant que nécessaire en fonction de la longueur du dispositif souhaitée.

Sur la figure 6, on peut dénombrer une succession de cinq tronçons munis de six viroles et de six ensembles de supportage et mis en rotation par cinq dispositifs d'entraînement.

L'appareil 101 est complété par un dispositif d'alimentation 120 du cylindre en matière première, constitué d'un moteur 121 entraînant un dispositif à vis sans fin 122 et recevant suivant la flèche 123 la matière première par une trémie 124.

L'extrémité de l'appareil 101 est complétée par un crible 122 constitué d'une tôle perforée assemblée par la virole 123 au dernier tronçon de l'appareil. La matière traitée qui passera à travers le crible sera évacuée sur une bande transporteuse représentée en 124. Le refus sera alors évacué sur un deuxième système à bande transporteuse 125.

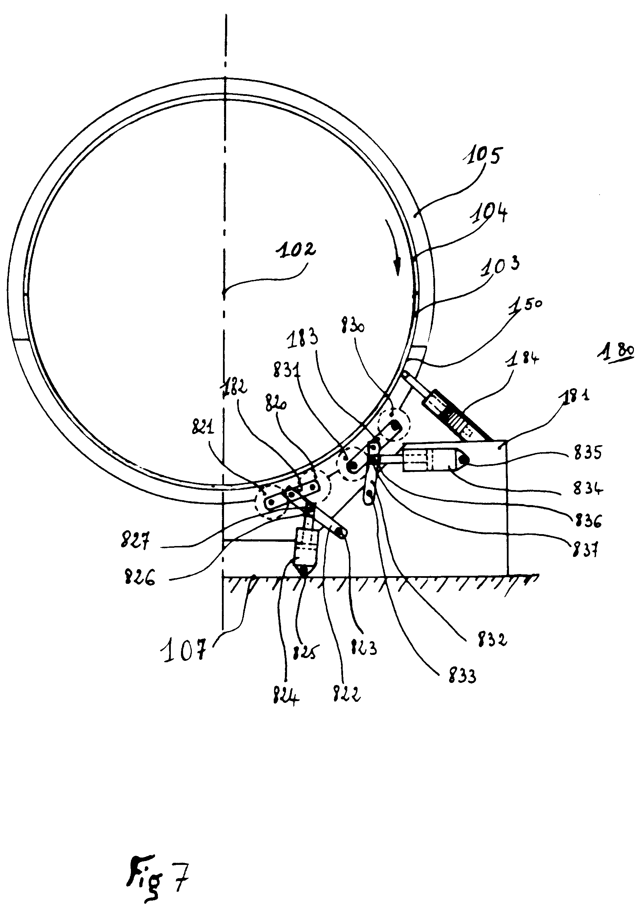

La figure 7 représente l'ensemble de supportage 180 en appui d'une part sur le sol en 107 et d'autre part sur la virole 105 elle-même soudée au tronçon 103. L'ensemble de supportage 180 est plus particulièrement constitué d'une structure métallique 181 sur laquelle sont disposées deux boggies 182, 183 constitués chacun d'une paire de galets de roulement respectivement 820, 821 et 830, 831, au contact par une de leur génératrice avec la surface courbe 150 du chemin de roulement. Le boggie 182 plus particulièrement appelé boggie de supportage est lié à un bras 822 monté pivotant par un axe 823 sur la structure métallique 181. Un vérin 824 est monté pivotant sur la structure 181 par l'intermédiaire d'un axe 825 et l'extrémité supérieure de son piston est liée au bras 822 par un étrier 826 et un axe 827. L'axe 827 est plus particulièrement situé entre l'axe 823 et le boggie 182 de manière à pouvoir déplacer le bras 822 par pivotement autour de l'axe 823.

Le boggie 183 plus particulièrement appelé boggie de centrage est porté par un bras 832 monté pivotant sur un axe 833 tandis qu'un vérin 834 monté pivotant sur un axe 835 est relié par l'intermédiaire d'un étrier 836 et d'un axe 837 à ce même bras 832. Cet axe 837 sera situé entre le boggie et l'axe 833 de manière à pouvoir faire pivoter le bras 832 autour de ce même axe 833.

Un capteur de déplacement 184 par exemple du type inductif est fixé sur la structure métallique 181 et l'extrémité de ce capteur vient en appui sur le chemin de roulement 150. L'axe du capteur de déplacement est confondu avec une droite perpendiculaire à l'axe longitudinal du cylindre. Il est ainsi possible à partir des informations recueillies par le capteur de connaître les déplacements du tronçon 103 suivant l'axe vertical et l'axe horizontal. L'ensemble de supportage 180 est reproduit à l'identique et de façon symétrique par rapport à un plan vertical passant par l'axe de rotation de manière à ce que chaque chemin de roulement prenne appui sur deux boggies porteurs et deux boggies de centrage.

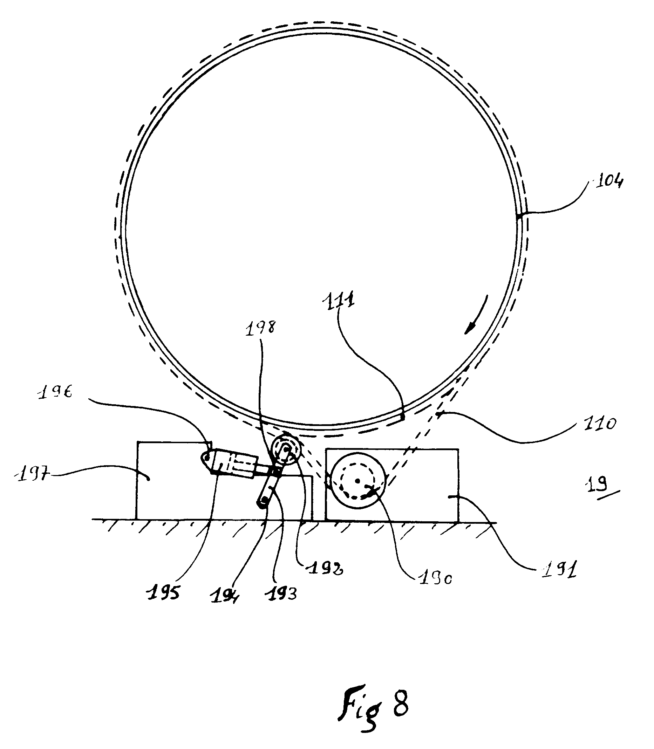

Le dispositif d'entraînement 109 est plus particulièrement représenté en figure 8 sur laquelle on peut voir la couronne dentée 111 et sa chaîne 110. Cette même chaîne 110 à la partie inférieure de l'appareil est mise en mouvement par le pignon 190 situé sur l'arbre du moteur hydraulique 191. Par ailleurs la chaîne passe également sur un galet tendeur monté libre sur un bras 193 lui-même monté pivotant sur un axe 194. L'extrémité du piston d'un vérin 195 lié par un axe 196 à la structure fixe 197 est montée sur un axe 198 situé entre le galet et l'axe 194.

Le système hydraulique non représenté est constitué de façon classique d'une centrale hydraulique fournissant un fluide à très haute pression, d'un ensemble d'électrovannes, pilotées par une centrale de pilotage du type calculateur. Ces électrovannes sont donc disposées entre la source de pression et les différents vérins. Le calculateur est également relié aux différents capteurs de déplacement.

Nous allons maintenant décrire le fonctionnement d'un tel appareil appliqué notamment à un cylindre pour le traitement par fermentation accélérée des ordures ménagères. Des ordures ménagères sont amenées à l'aide de moyens de transport dans la trémie 124 et sont distribuées à l'intérieur de l'appareil 101 grâce à la vis sans fin 122 mise en rotation par le moteur électrique 121. Le traitement de fermentation accéléré consiste à laisser fermenter les matières organiques contenues dans les ordures de façon à ce que la température s'élève aux environs de 70°C tout en assurant un brassage en continu. La longueur de l'appareil, la vitesse de rotation et l'inclinaison seront calculées de manière à ce que le temps de séjour des ordures soit de 48 heures minimum. Ainsi, on obtient un compost vert, stérilisé et sans odeur . Le criblage final aura pour but de retirer les matières non organiques telles que ferrailles, verres, et autres matières plastiques. La masse du produit à traiter varie tout au long de l'appareil en fonction de l'alimentation et de l'évacuation. Les différentes sections sont donc plus ou moins sollicitées. La multiplication des points de supportage permet de diminuer de façon considérable les contraintes mécaniques auxquelles chacun des tronçons est soumis. En effet, chaque tronçon est muni d'au moins un capteur de déplacement qui détecte tout déplacement du cylindre sous l'effet de la charge locale. Le déplacement est alors transmis au calculateur qui interprète cet élément pour délivrer un signal électrique à l'électrovanne qui à son tour laisse passer une certaine quantité de fluide à très haute pression en direction de la chambre désignée du vérin associé ce qui a pour effet résultant de compenser la surcharge et de conserver la position initiale donc un parfait alignement. Un tel ensemble de supportage à compensation hydraulique permet également d'absorber les faux ronds et les défauts de concentricité des chemins de roulement. Ces divers composants ne demandent donc pas des usinages de haute précision en fabrication et le prix de revient en est diminué d'autant.

Le dispositif d'entraînement est également divisé en plusieurs sous-ensembles identiques pour chacun des tronçons.Le moteur hydraulique est alimenté par la même centrale de pression ainsi que les différents vérins y compris les vérins tendeurs.Ces différents moteurs hydrauliques et ces différents vérins tendeurs sont également munis d'électrovannes pilotées par le calculateur.En fonction des informations des capteurs de déplacement donc de la charge, la tension pourra être augmentée ou diminuée de manière à supprimer les différents effets de répulsion ou simplement faire varier le couple. Les avantages qui découlent de la mise en place des ensembles de supportage et des dispositifs d'entraînement selon l'invention conduisent à la réalisation d'un cylindre de fermentation dont l'épaisseur de la tôle est de 6 à 10 mm, constante tout au long de l'appareil et ceci indépendamment de la longueur dudit appareil. En effet, chaque tronçon rajouté sera muni d'un ensemble de supportage et d'entraînement indépendant qui sera simplement relié à la centrale hydraulique et à la centrale de pilotage. A l'heure actuelle, on estime qu'un tube de 40 mètres est capable de traiter une quantité d'ordures correspondant à environ 150 à 200 000 habitants.Dans les réalisations à supportage mécanique, des problèmes de torsions importants ou des coûts prohibitifs empêchaient la réalisation d'appareils de longueur supérieure à 60 mètres. On peut donc maintenant réaliser des tubes proportionnellement à la quantité d'ordures à traiter.

Dans une réutilisation avantageuse de la présente invention la paroi intérieure du cylindre 2 ou des tronçons 103, 104 est munie de pales ou d'ailettes comme cela est connu pour des mélangeurs de béton.

Ces pales ont surtout pour but d'améliorer le brassage des déchets mais, plus particulièrement, de permettre le déplacement des déchets dans le sens axial du cylindre.

Pour effectuer ce déplacement axiale des déchets dans l'orientation et avec la vitesse désirées, les pales sont supportées de façon orientable dans le but de produire un effet d'entraînement lors de leur rotation avec le cylindre, dans le sens général d'acheminement du contenu de l'appareil 1 ou dans le sens inverse afin de contrôler la répartition de la charge le long du cylindre par orientation individuelle des pales disposées à plusieurs endroits dans le sens axial.

On peut aussi prévoir de faire tourner le cylindre 1 en laissant ouverte sa porte arrière afin de permettre le libre passage d'air en orientant les dernières pales de façon à repousser le contenu du cylindre vers son intérieur. Ce mode de fonctionnement permet un réglage efficace de la température de la matière traitée.

L'utilisation des pales comme décrit ci-dessus permet aussi un chargement du cylindre plus efficace, car les déchets placés dans l'extrémité d'entrée de ce dernier sont rapidement répartis sur toute la longueur du cylindre.

Le réglage des pales peut être commandé automatiquement par la centrale de pilotage en fonction des valeurs mesurées,telles que température,humidité, degré de chargement etc., et ce réglage peut être effectué individuellement pour chaque pale ou pour leur ensemble.

Les pales et leur orientation sont donc utilisées pour contrôler la vidange du cylindre, et la régularisation du niveau des produits qu'il renferme. Elles facilitent le remplissage rapide du cylindre pendant les heures de travail et régularisent le niveau, par exemple, pendant les périodes de repos.

La présente invention n'est pas limitée au mode particulier de réalisation qui vient d'être décrit, c'est ainsi que l'on peut mesurer d'autres paramètres tels que le pH du milieu, le taux d'oxygène.

Le coefficient de remplissage peut également être déterminé par les mesures de la pression dans les dispositifs de supportage à compensation de pression.

La transmission des informations est réalisable par fibres optiques et interface optique sans contact.

L'ensemble du système hydraulique pourrait être remplacé par un système pneumatique.Le traitement des différentes informations et le pilotage pourraient également être effectués à l'aide d'une carte analogique. Le chemin de roulement en U peut également être remplacé par un profilé en T, la partie horizontale faisant fonction de chemin de roulement et la largeur étant calculée pour tenir compte des dilatations éventuelles de l'appareil.

## Revendications

1. Dispositif de traitement de déchets, notamment d'ordures ménagères, du type comprenant un cylindre de fermentation (101) à axe de rotation sensiblement horizontal dans lequel la charge en déchets peut varier suivant la longueur, et plusieurs dispositifs de supportage (106, 108) repartis le long de l'axe dudit cylindre, et comportant chacun un moyen de réglage de la pression de supportage ( 824, 834), caractérisé en ce qu'un dispositif de mesure de la charge du cylindre (184) est prévu au droit de chaque dispositif de supportage (106, 108), et en ce que le moyen de réglage de pression de ce dispositif de supportage est commandé en fonction des indications dudit dispositif de mesure.

2. Dispositif selon la revendication 1, caractérisé en ce qu'il comprend plusieurs moyens moteurs (110, 111, 190 à 198) pour entraîner positivement le cylindre en rotation en chacun de plusieurs points espacés axialement les uns des autres.

3. Dispositif selon la revendication 2, caractérisé en ce que chaque moyen moteur comprend un moteur hydraulique (191) relié audit cylindre par une chaîne d'entraînement (110) munie d'un galet tendeur hydraulique (192 à 196).

4. Dispositif selon la revendication 3, caractérisé en ce que le galet tendeur hydraulique (192 à 196) est commandé en fonction des indications d'un dispositif de mesure de la charge du cylindre (184).

5. Dispositif selon l'une des revendications 3 et 4, caractérisé en ce que le couple moteur du moteur hydraulique (191) est commandé en fonction des indications d'un dispositif de mesure de la charge du cylindre (184).

6. Dispositif selon l'une des revendications 2 à 5, caractérisé en ce que chaque moyen moteur (110, 111, 190 à 198) est associé à un dispositif de supportage (106, 108) et qu'il est situé à proximité de ce dernier.

7. Dispositif selon la revendication 7, caractérisé en ce qu'il comprend des moyens pour la mesure de la température (11), du degré hygrométrique (27, 33, 34) de la quantité de produit (50) et pour la régulation (44, 45).

8. Dispositif selon la revendication 7, caractérisé en ce qu'il comporte un microprocesseur (12) embarqué solidaire du cylindre (2) en liaison avec une centrale de pilotage (16), les moyens de mesure (11, 27, 33, 34, 50) et de régulation (44, 45).

9. Dispositif selon la revendication 8, caractérisé en ce que les moyens de régulation sont constitués de deux piquages (44, 45) alimentés en eau (42) et air (43).

10. Dispositif selon l'une des revendications 7 à 9, caractérisé en ce que la sonde de mesure de température (11) est disposée à l'intérieur de dents (10) de déchiquetage solidaires de l'appareil.

11. Dispositif selon l'une des revendications 7 à 10, caractérisé en ce que le moyen de mesure du degré hygrométrique comprend une sonde (27) disposée dans une enceinte (22) d'échantillonnage isolée par les électrovannes (23, 24) et remplie par le ventilateur d'aspiration (26) grâce à la prise d'aspiration (20).

12. Dispositif selon l'une des revendications 7 à 10, caractérisé en ce que le moyen de mesure du degré hygrométrique comprend deux électrodes (33, 34) positionnées avec un écartement fixe sur un support (32) lui-même solidaire de la paroi (2).

13. Dispositif selon l'une des revendications 7 à 12, caractérisé en ce que le moyen de mesure de la quantité de produit comporte un microphone (52) placé dans une enceinte isolée (51) associée à un codeur angulaire (55).

14. Dispositif selon l'une des revendications précédentes, caractérisé en ce que chaque dispositif de supportage comprend des galets de supportage (820, 821) et des galets de centrage (830, 831).

15. Dispositif selon la revendication 14, caractérisé en ce que lesdits galets sont disposés de façon symétrique de part et d'autre du plan vertical passant par l'axe de révolution (102) de l'appareil.

16. Dispositif de supportage selon la revendication 15, caractérisé en ce que chaque dispositif de mesure de la charge du cylindre comprend au moins deux capteurs (184) disposés de part et d'autre du plan vertical passant par l'axe de révolution (102) de l'appareil.

17. Dispositif de supportage selon l'une des revendications précédentes, caractérisé en ce qu'il comprend un calculateur disposé en interface entre les différents capteurs et moyens de réglage de pression (824, 836) des dispositifs de supportage (106, 108).

18. Dispositif selon la revendication 17, caractérisé en ce que les moyens de réglage de pression comprennent des vérins hydrauliques (824, 834) commandés par une centrale hydraulique et en ce que le calculateur agit directement sur des électrovannes elle-mêmes disposées entre la centrale hydraulique et les différents vérins hydrauliques (824, 834).

19. Dispositif selon l'une des revendications précédentes, caractérisé en ce qu'il comporte des moyens pour mélanger les produits qu'il contient, constitués par des ailettes montées sur la paroi intérieure du cylindre de façon orientable, et en ce que leurs orientations sont contrôlées individuellement ou généralement par le dispositif de pilotage.

## Claims

1. Apparatus for the treatment of refuse, in particular household waste, of the type comprising a fermentation cylinder (101) having a substantially horizontal axis of rotation in which the load of refuse can vary along its length, and a plurality of support devices (106, 108) spaced apart along the axis of said cylinder, and each comprising means for controlling the support pressure (824, 834), characterized in that means for measuring the load of the cylinder (184) is provided at the position of each support device, (106, 108) and in that the means for controlling the pressure of this support device is actuated as a function of the indication of said measurement device.

2. Apparatus according to claim 1, characterized in that it comprises a plurality of motor means (110, 111, 190 to 198) for driving the cylinder in rotation positively at each of a plurality of points spaced axially one from another.

3. Apparatus according to claim 2, characterized in that each motor means comprises a hydraulic motor (191) connected to said cylinder by a drive chain (110) provided with a hydraulic roller tensioner (192 to 196).

4. Apparatus according to claim 3, characterized in that the hydraulic roller tensionner (192 to 196) is controled as a function of the indications from a device for measuring the load of the cylinder (184).

5. Apparatus according to one of claims 3 and 4, characterized in that the drive torque of the hydraulic motor (191) is controled as a function of the indications from a device for measuring the load of the cylinder (184).

6. Apparatus according to one of claims 2 to 5, characterized in that each motor means (110, 111, 190 to 198) is associated with a support device (106, 108) and that it is situated in proximity to the latter.

7. Apparatus according to claim 7, characterized in that it comprises means for measurement of the temperature (11), the degree of hygrometry (27, 33, 34) and the quantity of product (50) and for regulation (44, 45).

8. Apparatus according to claim 7, characterized in that it comprises an on-board microprocessor (12) solid with the cylinder (2) and connected with a control unit (16), the measurement means (11, 27, 33, 34, 50) and the regulation means (44, 45).

9. Apparatus according to claim 8, characterized in that the regulation means consists of two ports (44, 45) supplied with water (42) and air (43).

10. Apparatus according to one of claims 7 to 9, characterized in that the temperature measurement probe (11) is disposed within shredding teeth (10) solid with the apparatus.

11. Apparatus according to one of claims 7 to 10, characterized in that the means for measuring the degree of hygrometry comprises a probe (27) disposed within a sampling enclosure (22) which is isolated by electrovalves (23, 24) and filled by a suction fan (26) by means of a suction connection (20).

12. Apparatus according to one of claims 7 to 10, characterized in that the means for measuring the degree of hygrometry comprises two electrodes (33, 34) positioned with a fixed spacing on a support (32) which itself is solid with the wall (2).

13. Apparatus according to one of claims 7 to 12, characterized in that the means for measuring the quantity of product comprises a microphone (52) placed in an isolated enclosure (51) associated with an angular encoder (55).

14. Apparatus according to one of preceding claims, characterized in that each support means comprises support rollers (820, 821) and centering rollers (830, 831).

15. Apparatus according to claim 14, characterized in that said rollers are disposed symmetrically one on each side of the vertical plane through the axis of revolution (102) of the apparatus.

16. Support apparatus according to claim 15, characterized in that each device for measuring the load of the cylinder comprises at least two sensors (184) disposed on opposite sides of the vertical plane through the axis of revolution (102) of the apparatus.

17. Support apparatus according to one of the preceding claims, characterized in that it comprises a calculator disposed as interface between the different sensors and means for controlling the pressure (824, 826) of the support devices (106, 108).

18. Apparatus according to claim 17, characterized in that the means for controling pressure comprise hydraulic jacks (824, 834) actuated by a hydraulic unit and in that the calculator acts directly on electrovalves which are disposed between the hydraulic unit and the different hydraulic jacks (824, 834).

19. Apparatus according to one of the preceding claims, characterized in that it comprises means for mixing the products it contains, consisting of flaps mounted orientably on the interior wall of the cylinder, and in that the orientations are controlled individually or generally by the control device.

## Patentansprüche

1. Einrichtung zur Behandlung von Abfallstoffen, insbesondere von Hausmüll, bestehend aus einer Gärungstrommel (101) mit in etwa Waagerechter Drehachse, die eine Abfallbefüllung mit unterschiedlicher Länge aufnehmen kann, sowie aus mehreren Stützvorrichtungen (106, 108), die entlang der Achse der besagten Trommel verteilt sind und die jeweils ein Element für die Einstellung des Stützdrucks (824, 834) aufweisen, dadurch gekennzeichnet, daß eine Meßvorrichtung für die Trommelbefüllung (184) an jeder Stützvorrichtung (106, 108) Vorgesehen ist und daß das Element für die Druckeinstellung dieser Stützvorrichtung in Abhängigkeit von den Angaben der Meßvorrichtung betätigt wird.

2. Einrichtung nach Anspruch 1, dadurch gekennzeichnet, daß sie mehrere Antriebselemente (110, 111, 190 bis 198) für den positiven Drehantrieb der Trommel an jeder von mehreren Stellen aufweist, die axial in einem bestimmten Abstand voneinander angeordnet sind.

3. Einrichtung nach Anspruch 2, dadurch gekennzeichnet, daß jedes Antriebselement einen Hydraulikmotor (191) umfaßt, der über eine Antriebskette (110) mit einer hydraulischen Spannrolle (192 bis 196) an die Trommel angeschlossen ist.

4. Einrichtung nach Anspruch 3, dadurch gekennzeichnet, daß die hydraulische Spannrolle (192 bis 196) in Abhängigkeit von den Angaben einer Meßvorrichtung für die Trommelbefüllung (184) betätigt wird.

5. Einrichtung nach einem der Ansprüche 3 und 4, dadurch gekennzeichnet, daß das Antriebsmoment des Hydraulikmotors (191) in Abhängigkeit von den Angaben einer Meßvorrichtung für die Trommelbefüllung (184) geregelt wird.

6. Einrichtung nach einem der Ansprüche 2 bis 5, dadurch gekennzeichnet, daß jedes Antriebselement (110, 111, 190 bis 198) einer Stützvorrichtung (106, 108) zugeordnet ist und daß es in deren unmittelbarer Nähe angeordnet ist.

7. Einrichtung nach Anspruch 6, dadurch gekennzeichnet, daß sie Vorrichtungen für die Messung der Temperatur (11), des Feuchtigkeitsgehalts (27, 33, 34), der Produktmenge (50) und für die Regelung (44, 45) aufweist.

8. Einrichtung nach Anspruch 7, dadurch gekennzeichnet, daß sie einen fest an der Trommel (2) angebrachten Mikroprozessor (12) aufweist, der mit einer Steuereinheit (16), mit den Meßvorrichtungen (11, 27, 33, 34, 50) und mit den Regelvorrichtungen (44, 45) verbunden ist.

9. Einrichtung nach Anspruch 8, dadurch gekennzeichnet, daß die Regelvorrichtungen aus zwei Anschlüssen (44, 45) mit Wasser- (42) und Luftzuleitung (43) besteht.

10. Einrichtung nach einem der Ansprüche 7 bis 9, dadurch gekennzeichnet, daß der Temperaturmeßfühler (11) im Innern von Reißzähnen (10) angebracht ist, die einstückig mit dem Gerät ausgeführt sind.

11. Einrichtung nach einem der Ansprüche 7 bis 10, dadurch gekennzeichnet, daß der Feuchtigkeitsmesser einen Meßfühler (21) aufweist, der in einem Prüfgehäuse (22) angeordnet ist, das durch die Elektroventile (23, 24) isoliert und durch das Sauggebläse (26) über den Sauganschluß (20) befüllt wird.

12. Einrichtung nach einem der Ansprüche 7 bis 10, dadurch gekennzeichnet, daß der Feuchtigkeitsmesser zwei Elektroden (33, 34) aufweist, die mit einem festen Abstand auf einer Halterung (32) angeordnet sind, welche wiederum einstückig mit der Wand (2) ausgeführt ist.

13. Einrichtung nach einem der Ansprüche 7 bis 12, dadurch gekennzeichnet, daß die Meßvorrichtung für die Produktmenge ein Mikrophon (52) aufweist, das in einem isolierten Gehäuse (51) angebracht ist, welches einem Winkelcodierer (55) zugeordnet ist.

14. Einrichtung nach einem der vorangehenden Ansprüche, dadurch gekennzeichnet, daß jede Stützvorrichtung Stützrollen (820, 821) und Zentrierrollen (830, 831) umfaßt.

15. Einrichtung nach Anspruch 14, dadurch gekennzeichnet, daß die besagten Rollen symmetrisch auf beiden Seiten der Lotebene angeordnet sind, die durch die Drehachse (102) des Geräts verläuft.

16. Stützvorrichtung nach Anspruch 15, dadurch gekennzeichnet, daß jede Meßvorrichtung für die Trommelbefüllung mindestens zwei Meßfühler (184) umfaßt, die auf beiden Seiten der Lotebene angeordnet sind, die durch die Drehachse (102) des Geräts verläuft.

17. Stützvorrichtung nach einem der vorangehenden Ansprüche, dadurch gekennzeichnet, daß sie einen Rechner umfaßt, der in Form einer Schnittstelle zwischen den verschiedenen Meßfühlern und Druckreglern (824, 836) der Stützvorrichtungen angeordnet ist.

18. Einrichtung nach Anspruch 17, dadurch gekennzeichnet, daß die Druckregler hydraulische Stellglieder (824, 834) umfassen, die durch eine Hydraulikeinheit betätigt werden, und daß der Rechner direkt Elektroventile ansteuert, die wiederum zwischen der Hydraulikeinheit und den verschiedenen hydraulischen Stellgliedern (824, 834) angeordnet sind.

19. Einrichtung nach einem der vorangehenden Ansprüche, dadurch gekennzeichnet, daß sie Vorrichtungen für das Mischen der in ihr enthaltenen Produkte aufweist, die aus Verstellbar an der Innenwand der Trommel angebrachten Schaufeln bestehen, und daß deren Verstellung einzeln oder insgesamt durch die Steuereinheit kontrolliert wird.
